# EUROPEAN PATENT SPECIFICATION

(11) **EP 4 021 391 B1**
(45) Date of publication and mention of the grant of the patent: **17.01.2024**
(21) Application number: 20764956.7
(22) Date of filing: 21.08.2020
(51) Int. Cl.: A61K 8/41, A61Q 19/02, C07C 217/74, C07C 217/58

(54) **NOVEL COMPOUNDS FOR SKIN LIGHTENING**
NEUARTIGE VERBINDUNGEN ZUR HAUTAUFHELLUNG
NOUVEAUX COMPOSÉS POUR ÉCLAIRCIR LA PEAU

(30) Priority: 28.08.2019 EP 19193956
(43) Date of publication of application: 06.07.2022
(73) Proprietor: Unilever Global IP Limited, Wirral, Merseyside CH62 4ZD (GB); Unilever IP Holdings B.V., 3013 AL Rotterdam (NL)
(72) Inventor: ROSA, Jose, Guillermo, Trumbull, Connecticut 06611 (US)
(74) Representative: James, Helen Sarah
(86) International application number: PCT/EP2020/073541
(87) International publication number: WO 2021/037750

(56) References cited:
- WO-A1-2006/097223
- WO-A2-96/12697
- WO-A2-2010/097480
- US-A1- 2012 027 699
- REGISTRY: "4-((1-(4-(1-methylethoxy)phenyl)ethyl)ami no)cyclohexanol", UKRORGSYNTHESIS, 7 June 2009 (2009-06-07), XP55731826,
- REGISTRY: "4-((1-(4-(1-methylethoxy)phenyl)ethyl)ami no-cyclohexanol (trans)", AURORA FINE CHEMICALS, 15 May 2016 (2016-05-15), XP55731823,

## Description

### Field of the invention

The present invention relates to novel compounds for skin reduction in hyperpigmentation.

### Background of the invention

Cosmetic compositions of various kinds are widely used by consumers. Skin care cosmetics such as lotions and creams are applied to obtain benefits like e.g. anti-aging, skin reduction in hyperpigmentation and moisturizing.

Skin, the outermost protective covering of living beings, is more susceptible to get affected by exposure to factors like e.g. sunlight, heat, humidity, pollution and dust. Overexposure to these factors may lead to conditions like e.g. tanning, blotchy skin, hyperpigmentation, freckles, melasma, which may in turn lead to e.g. less preferred uneven skin tones.

One of the possible ways to reduce such conditions is by ensuring minimal exposure to the affecting factors mentioned above, particularly sunlight. It is generally said that overexposure to sunlight and thereby to harmful ultraviolet rays contained therein, gives rise to a tanning effect. However, ensuring minimal exposure to such factors alone is not always sufficient and in most cases, exposure to such factors and particularly to sunlight, is unavoidable.

It is for these reasons, people have been relying on use of cosmetics to reduce hyperpigmentation of skin and/or even skin tones. Skin lightening agents are well known in the art. However, many of the known substances like e.g. kojic acid, tend to have either low efficacy or may cause undesirable side effects such as skin irritation. Therefore, alternative agents that for e.g. will deliver better reduction of skin hyperpigmentation and/or no or low side effects are much desired.

WO 99/04752 (Johnson and Johnson) discloses a method and a composition for providing changes in mammalian skin pigmentation that comprised a topical application of a compound which acts through PAR-2 pathway. Particularly, it disclosed compounds that act as trypsin, tryptase, serine protease or as PAR-2 agonists for increase in pigmentation. And, trypsin inhibitors, thrombin inhibitors, tryptase inhibitors as PAR-2 pathway inhibitors or as PAR-2 antagonists for depigmentation.

WO 98/56757 and JP2000178188 (both by Sankyo Co) disclose benzylamine derivatives through a general formula given therein. The compound and its pharmaceutically acceptable salts have been shown to have excellent ileal bile acid transporter inhibitory activity.

A new class of compounds according to formula 1a, 1b or 1c or cosmetically acceptable salts thereof has now been found. These compounds have been found to deliver skin reduction in hyperpigmentation effect. Further, these compounds have been found to deliver a good efficacy in skin reduction in hyperpigmentation and/or no or low side effects, like e.g. skin irritation.

WO 2010 097 480 describes substituted phenylaminocyclohexyl derivatives as skin whitening agents but having a mandatory carbamate linker between the cyclohexyl and amino groups.

### Summary of the invention

In a first aspect, the present invention provides a novel compound or cosmetically acceptable salts thereof, as described in claim 1.

In a second aspect, the present invention provides a cosmetic method of reducing age spots and freckles comprising the step of applying a composition according to the present invention that contains, a compound of formula 1 or cosmetically acceptable salt thereof.

### Definitions

As used herein, "salts" mean halogen salts, tosylate salts, mesylate salts, sulfate salts, phosphate salts, citrate salts, tartrate salts, linear, branched or cyclic carboxylates and dicarboxylates salts; in particular C₂ to C₁₂ alkylcarboxylates; which can be saturated or unsaturated and substituted with heteroatoms selected from oxygen, along with any other counter ions used in the cosmetic industry.

Unless specified otherwise, amounts as used herein are expressed in percentage by weight based on total weight of the composition and is abbreviated as "wt%".

As used herein "lightening" means reducing hyperpigmentation of the skin, like age spots and freckles.

### Detailed description of the invention

The present invention provides a compound according to formula 1a: 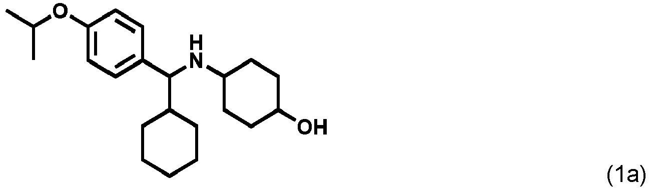 or formula 1b: 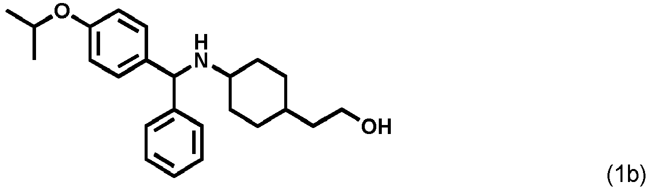 or formula 1c: 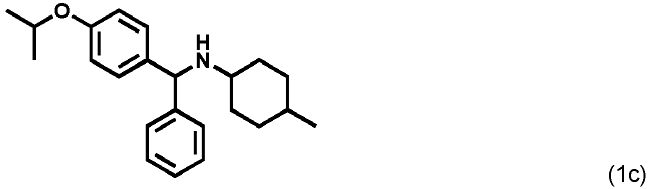 or cosmetically acceptable salt thereof.

Compounds according to the present invention have been found to deliver a reduction in skin hyperpigmentation.

### Composition according to the present invention

The present invention further concerns a composition comprising a compound of the present invention, i.e., a compound of formula 1a, 1b or 1c or cosmetically acceptable salts thereof, and cosmetically acceptable base.

The composition according to the present invention can be a composition for topical application to skin of mammals, especially humans. Such a composition may be generally classified as leave-on or rinse off, and is meant to include conditioners or tonics, lipsticks, colour cosmetics and general topical compositions.

The composition according to the present invention is preferably a leave-on composition.

The composition according to present invention comprises from 0.001 to 20 wt% preferably from 0.01 to 15 wt%, more preferably from 0.1 to 10 wt%, even more preferably from 0.5 to 5 wt% and most preferably from 1 to 3 wt% of a compound of formula 1 or cosmetically acceptable salts thereof.

### Cosmetically acceptable base

The composition comprises a cosmetically acceptable base to act as a diluent, dispersant or carrier for other materials present in the composition, so as to facilitate their distribution when the composition is applied to the skin.

Cosmetically acceptable bases include fatty acids having from 10 to 30 carbon atoms and salts thereof, water, liquid or solid emollients, solvents, humectants, thickeners and powders, skin penetration enhancers and can be used alone or as mixtures thereof.

Illustrative examples of fatty acids having from 10 to 30 carbon atoms include pelargonic, lauric, myristic, palmitic, stearic, isostearic, oleic, linoleic, arachidic, behenic or erucic acid, and mixtures thereof. An illustrative example of salts of fatty acid is potassium stearate.

Illustrative examples of emollients include stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, din-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate and myristyl myristate.

Illustrative examples of solvents include ethyl alcohol, isopropanol, acetone, ethylene glycol monoethyl ether, diethylene glycol monobutyl ether and diethylene glycol monoethyl ether.

Illustrative examples of powders include chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose and ethylene glycol monostearate.

Compounds that are believed to enhance skin penetration, like dimethyl sulfoxide, may also be used as cosmetically acceptable base.

Preferred bases are water, stearic acid, potassium stearate and mixtures thereof.

The cosmetically acceptable base is usually present from 10 to 99.9 wt%, preferably from 50 to 99 wt% and can form the balance of the composition.

The composition comprising a compound according to the present invention may further comprise additional skin lightening agents.

### Additional skin lightening agents

Illustrative examples of additional skin lightening agents include vitamin B3 compounds, vitamin B6, vitamin C, vitamin A, resorcinol derivatives, 12-hydroxystearic acid, glutathione precursors, galardin, adapalene, aloe extract, ammonium lactate, arbutin, azelaic acid, butyl hydroxy anisole, butyl hydroxy toluene, citrate esters, deoxyarbutin, 1,3-diphenylpropane derivatives, 2,5-dihydroxybenzoic acid and its derivatives, 2-(4-acetoxyphenyl)-1,3-dithiane, 2-(4-hydroxyphenyl)-1,3-dithiane, ellagic acid, gluco pyranosyl-1-ascorbate, gluconic acid, glycolic acid, green tea extract, 4-Hydroxy-5-methyl-3[2H]-furanone, hydroquinone, 4-hydroxyanisole and its derivatives, 4-hydroxy benzoic acid derivatives, hydroxycaprylic acid, inositol ascorbate, kojic acid, lactic acid, lemon extract, linoleic acid, magnesium ascorbyl phosphate, 5-octanoyl salicylic acid, , salicylic acid, 3,4,5-trihydroxybenzyl derivatives, octadecenedioic acid, acetylglucosamine, pitera extract, symwhite, calcium pantothenate (Melano-block), seppiwhite, soybean extract (bowman birk inhibitor) and mixtures thereof.

Preferred skin lightening agents are vitamin B3 compounds i.e. niacin, niacinamide, nicotinyl alcohol, or derivatives or salts thereof, vitamin B6, resorcinol derivatives i.e. 2,4-substituted resorcinol derivatives, 3,5-substituted resorcinol derivatives, hexylresorcinol and phenylethyl resorcinol, 12-hydroxystearic acid, glutathione precursors and galardin.

When incorporated in the composition, an additional skin lightening agent is added preferably from 0.001 to 15 wt%, more preferably from 0.01 to 10 wt% and most preferably from 0.1 to 5 wt%.

The composition according to the present invention may include a combination of compound of formula 1a, 1b or 1c with at least one compound selected from niacinamide, 12- hydroxystearic acid, glutathione precursors, resorcinol derivatives, in particular hexyl resorcinol, octadecenedioic acid, acetylglucosamine, pitera extract, symwhite, calcium pantothenate (Melano-block), seppiwhite and soybean extract (bowman birk inhibitor).

### Sunscreens

### Organic Sunscreens

The composition preferably additionally comprises one or more organic sunscreens. A wide variety of organic sunscreens is suitable for use in compositions of this invention.

Suitable UV-A / UV-B sunscreens include, 2-hydroxy-4-methoxybenzophenone, octyldimethyl p-aminobenzoic acid, digalloyltrioleate, 2,2-dihydroxy-4-methoxybenzophenone, ethyl-4-(bis(hydroxypropyl))aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl-p-dimethylaminobenzoate, 2-phenylbenzimidazole-5-sulfonic acid, 2-(p-dimethylaminophenyl)-5-sulfonicbenzoxazoic acid, 2-ethylhexyl-p-methoxycinnamate, dibenzoylmethane derivatives, 2-hydroxy-4-methoxybenzophenone, octyldimethyl-p-aminobenzoic acid, diethylhexyl naphthylate, Mexoryl, Tinosorb S, Tinosorb M and mixtures thereof.

Preferred dibenzoylmethane derivatives are 4-tert-butyl-4'-methoxydibenzoylmethane, 2-methyldibenzoylmethane, 4-methyldibenzoylethane, 4-isopropyldibenzoylmethane, 4-tert-butyldibenzoylmethane, 2,4-dimethyldibenzoylmethane, 2,5-dimethyldibenzoylmethane, 4,4'-diisopropyldibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane or 2,6-dimethyl-4-tert-butyl-4'-methoxydibenzoylmethane.

Preferred organic sunscreens are 2-ethylhexyl-p-methoxycinnamate (Parsol MCX), dibenzoylmethane derivative; in particular 4-tert.-butyl-4'-methoxydibenzoylmethane (Parsol 1789), 2-ethylhexyl-2-cyano-3,3-diphenyl-2-propenoate (Octocrylene) or mixtures thereof.

An effective amount of organic sunscreens may be used in the compositions of the present invention. The composition preferably comprises from 0.1 to 15 wt%, more preferably from 1 to 10 wt%, most preferably from 2 to 5 wt% organic sunscreens.

### Inorganic Sunscreens

The composition may further comprise inorganic sunscreens. Illustrative examples of inorganic sunscreens are zinc oxide, iron oxide, silica, such as fumed silica, or titanium dioxide.

Preferred inorganic sunscreens are titanium dioxide (TiO2) and zinc oxide (ZnO).

The composition preferably comprises from 0.1 to 15 wt%, more preferably from 1 to 10 wt%, most preferably from 2 to 5 wt% an inorganic sunscreens.

### Method of skin lightening

The present invention further concerns a method of lightening the skin of a human. The method comprises the step of applying the composition comprising a compound according to the invention onto the human skin.

### Optional cosmetic ingredients

The compositions of the present invention can comprise a wide range of other optional components. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

### Product form

A composition according to the present invention is preferably formulated in the form of a powder, flake, lotion, cream, gel or mousse.

### Synthesis of compounds according to the present invention

Compounds of the present invention of Formula 1a, 1b or 1c can be classified as benzylamine derivatives. Numerous examples of benzylamines derivatives with basic characteristics to inventive compounds of Formula 1a, 1b or 1c have been synthesized using standard synthetic transformations and methods known to anyone skilled in the art, for example via reductive amination of ketone derivatives or amination of alkyl(aryl)halomethanes or diphenylhalomethane derivatives. The following prior art describes in detail the standard synthetic transformations and methodology available to anyone skilled in the art to allow the preparation of the inventive compounds of Formula 1: R.W. Hanson and H.D. Law (1965) "Substituted diphenylmethyl protecting groups in peptide synthesis" Journal of the Chemical Society, p. 7285-7297 (several examples described); V. Lazov, T. et al. (1999) "Laboratory method for synthesis of N-methyl-N-(diphenylmethyl)cyclohexylamine" Farmatsiya, 46, p. 11-12; N. Toda et al. (2003) "Design, synthesis and structure-activity relationships of dual inhibitors of acetylcholinesterase and serotonin transporter as potential agents for Alzheimer's disease" Bioorganic and Medicinal Chemistry, 11, 1935-1955 (compounds 13, 14a-g, 27 and 28); D.L.J. Clive et al. (2003) "Derivatized amino acids relevant to native peptide synthesis by chemical ligation and acyl transfer" Journal of Organic Chemistry, 68, 9247-9254 (several examples described); R. Sheng et al. (2005) "Design, synthesis and evaluation of 2-phenoxy-indan-1-one derivatives as acetylcholinesterase inhibitors" Bioorganic and Medicinal Chemistry, 15, 2834-2837 (compounds 4a,b and 5a-I); X.-H. Cai et al. (2006) "Methyl 2-methoxycarbonyl-3-phenylpropionate derivatives: A new type of angiotensin converting enzyme inhibitors" Letters in Organic Chemistry, 3, 492-494 (compounds 6, 7 and 8, page 493); J. Cherian (2011) "Structure-activity relationships of antitubercular nitroimidazoles. 3. Exploration of the linker and lipophilic tail of ((S)-2-nitro-6,7-dihydro-5H-imidazo[2,1-b][1,3]oxazin-6-yl)-(4-trifluoromethoxybenzyl)amine (6-Amino PA-824)" Journal of Medicinal Chemistry, 54, 5639-5659 (compounds 14a-c, 15a-c and 17a-i); C. Malavasic et al. (2011) "Synthesis and structure of novel (S)-1,6-dialkylpiperazine-2,5-diones and (3S,6S)-1,3,6-trialkylpiperazine-2,5-diones" Tetrahedron:Asymmetry, 22, 629-640 (compound 2c); S.E. Hampton et al. (2011) "Design, synthesis and evaluation of 5'-diphenyl nucleoside analogues as inhibitors of the Plasmodium falciparum dUTPase" ChemMedChem, 6, 1816-1831 (compounds 16d-f); O. Rahman et al. (2004) "Synthesis of [11C]/(113C)amines via carbonylation followed by reductive amination" Organic and Biomolecular Chemistry, 2, 1612-1616 (compounds 21a-k); A.R. Hajipour et al. (2001) "Butyltriphenylphosphonium tetraborate (BTPPTB) as a selective reducing agent for the reduction of imines, enamines and oximes and reductive alkylation of aldehydes or ketones with primary amines in methanol or under solid-phase conditions" Indian Journal of Chemistry, Section B: Organometallic Chemistry Including Medicinal Chemistry, 40B, 152-156; C. Salmi et al. (2006) "Efficient diastereoselective titanium(IV) reductive amination of ketones" Letters in Organic Chemistry, 3, 384-389; K. Hitoshi et al. (2001) "Preparation of cyclobutene derivatives as bile acid transporter inhibitors" EP 1070703 A1 (several examples described); D. Seebach et al. (1985) "Enantioselective addition of aryl groups to aromatic aldehydes using chiral aryltitanium binaphthol derivatives" Chemische Berichte, 118, 3673-3682 (compounds 4 and 5); F. Gasparrini et al. (1988) "Nitric acid facile oxidation of mono- and diarylcarbinols to carbonyl compounds in a biphasic system" Synthetic Communications, 18, 69-75; J. Wang et al. (1989) "Studies on enantioselective addition of chiral titanium reagents to aromatic aldehydes" Synthesis, 291-292; Wei-Sheng Huang and Lin Pu (1999) "The first highly enantioselective catalytic diphenylzinc additions to aldehydes: synthesis of chiral diarylcarbinols by asymmetric catalysis" Journal of Organic Chemistry, 64, 4222-4223; Chao-Jun Li and Yue Meng (2000) "Grignard-type carbonyl phenylation in water and under an air atmosphere" Journal of the America Chemical Society, 122, 9538-9539 (several compounds described); Alois Fuerstner and Helga Krause (2001) "Practical method for the rhodium-catalyzed addition of aryl- and alkenylboronic acids to aldehydes" Advanced Synthesis & Catalysis, 343, 343-350 (several examples described); H. Pajouhesh et al. (2010) "Structure-activity relationships of diphenylpiperazine N-type calcium channel inhibitors" Bioorganic and Medicinal Chemistry Letters, 20, 1378-1383; J. Belzner et al. (1989) "Synthesis of [1.1.1.]propellanes" Chemische Berichte, 122, 1509-1529; T. Kolasa et al. (2000) "Heteroarylmethoxyphenylalkoxyiminoalkylcarboxylic acids as leukotriene biosynthesis inhibitors" Journal of Medicinal Chemistry, 43, 690-705 (compounds 86, 101, 104 and 138); G.W. Kalbalka et al. (2001) "Alkylation of aromatic aldehydes with alkylboron chloride derivatives" Tetrahedron, 57, 1663-1670 (several examples described); C. Keh et al. (2003) "The Barbier-Grignard-type carbonyl alkylation using unactivated alkyl halides in water" Journal of the American Chemical Society, 125, 4062-4063 (several examples described); R. Apodaca et al. (2003) "A new class of diamine-based human histamine H3 receptor antagonists: 4-(aminoalkoxy)benzylamines" Journal of Medicinal Chemistry, 46, 3938-3944 (compound 14); S-B Qi et al. (2013) "Copper-dipyridylphosphine-catalyzed hydrosilylation: enantioselective synthesis of aryl- and heteroarylcycloalkyl alcohols" Organic and Biomolecular Chemistry, 11, 929-937; P. Averback et al. (1998) "Benzhydryl and bisbenzhydryl derivatives for the treatment of cerebral amyloidosis" WO 9852898 A1; L. Wang et al. (2002) "Synthesis and antiallergic activities of diphenylmethylpiperazine derivatives" Zhongguo Yaowu Huaxue Zazhi, 12, 125-129; T. Guzi et al. (2003) "Preparation of substituted 1-benzhydryl-4-[2-(4-piperidinyl)acetyl]-piperazines as 17-b-hydroxysteroid dehydrogenase type 3 inhibitors for the treatment of androgen dependent diseases" WO 2003022835 A1; Davood Setamdideh and Behzad Zeynizadeh (2006) "Mild and convenient method for reduction of carbonyl compounds with the NaBH4/charcoal system in wet THF" Zeitschrift fuer Naturforschung B: Chemical Sciences, 61, 1275-1281 (several examples described).

### Abbreviations for examples

ACN = acetonitrile
CHCl₃ = chloroform
d = days
DCM = dichloromethane
DIPEA = N,N-diisopropylethylamine
DMF = N,N-dimethylformamide
DMSO = dimethylsulfoxide
EA = ethyl acetate
EtOH = ethanol
FC = flash chromatography
g = gram
GC-MS = gas chromatography with mass spectrometry detection
HPLC-UV = high performance liquid chromatography with ultraviolet detection
¹H-NMR = proton nuclear magnetic resonance
IPA = isopropyl alcohol
K₂CO₃ = potassium carbonate
LC-MS = liquid chromatography with mass spectrometry detection
MeOH = methanol
MHz = megahertz
ul = microliter
ml = milliliter
mmol = millimole
NaBH₄ = sodium borohydride
NaCl = sodium chloride
NaOH = sodium hydroxide
Na₂SO₄ = sodium sulfate
PBS = phosphate buffer saline
PMA = phosphomolybdic acid
R.T. = room temperature
SOCl₂ = thionyl chloride
TEA = triethylamine
TFA = trifluoroacetic acid
THF = tetrahydrofuran
TLC = thin layer chromatography

The invention is further described using the following non-limiting examples.

### Examples

### Example 1: 4-((cyclohexyl(4-isopropoxyphenyl)methyl)amino)cyclohexanol

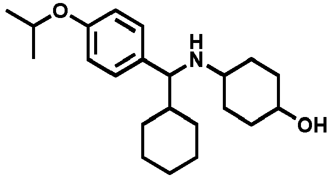

2-Bromopropane (30.2g, 246mmol) was added to a mixture of 4-hydroxybenzaldehyde (20g, 164mmol) and potassium carbonate (K₂CO₃) (45.3g, 328mmol) in N,N-dimethylformamide (DMF) (150ml) and the mixture stirred at 50 °C until complete disappearance of 4-hydroxybenzaldehyde as monitored by thin layer chromatography (TLC). The mixture was partitioned between ethyl acetate (EA) (300ml) and water (300ml) and the organic layer dried with sodium sulfate (Na₂SO₄), filtered and the solvents removed in vacuo to give crude 4-isopropoxybenzaldehyde (26g, 96%). A solution of crude 4-isopropoxybenzaldehyde (10g, 61mmol) in tetrahydrofuran (THF) (60ml) was added to a solution of cyclohexylmagnesium bromide (12.6g, 67.1mmol - prepared from cyclohexylbromide and magnesium) in THF (60ml) and the solution stirred at room temperature (R.T.) until consumption of 4-isopropoxybenzaldehyde as monitored by TLC. The mixture was partitioned between EA (200ml) and water (200ml) and the organic layer dried with Na₂SO₄, filtered and the solvents removed in vacuo to give cyclohexyl(4-isopropoxyphenyl)methanol which was purified by flash chromatography (FC) to give pure product as a colorless oil (6g, 40%). Methanesulfonyl chloride (3.1ml, 40mmol) was added to a solution of cyclohexyl(4-isopropoxyphenyl)methanol (6g, 36.5mmol) in dichloromethane (DCM, 60ml), followed by triethylamine (TEA) (5.9ml, 42mmol) and the solution stirred at room temperature until consumption of cyclohexyl(4-isopropoxyphenyl)methanol as monitored by TLC. The mixture was partitioned between EA (200ml) and water (200ml) and the organic layer dried with Na₂SO₄, filtered and the solvents removed in vacuo to give crude 1-(chloro(cyclohexyl)methyl)-4-isopropoxybenzene which was used for the next step without further purification (6g, 92%). 4-Aminocyclohexanol (126mg, 1.1mmol) was added to a solution of 1-(chloro(cyclohexyl)methyl)-4-isopropoxybenzene (300mg, 1.1mmol) in acetonitrile (ACN) (2ml) and the solution stirred at R.T. until consumption of 1-(chloro(cyclohexyl)methyl)-4-isopropoxybenzene as monitored by TLC. The mixture was partitioned between EA (200ml) and water (200ml) and the organic layer dried with Na₂SO₄, filtered and the solvents removed in vacuo to give crude 4-((cyclohexyl(4-isopropoxyphenyl)methyl)amino)cyclohexanol which was purified by FC to give pure product as a colorless oil which solidified on standing (140mg, 36%). HPLC-UV showed >99% purity. LC-MS (ESI⁺) showed expected mass [M+H]⁺ 346.5; ¹H NMR (400 MHz) δ 7.34 (2H, dd), 7.02 (2H, d), 4.67 (1H, m), 4.10 (1H, d), 3.51 (1H, m), 3.22 (1H, q), 2.68 (1H, m), 2.3-1.4 (10H, m), 1.31 (6H, d), 1.30-0.9 (8H, m).

### Example 2: 2-(4-(((4-isopropoxyphenyl)(phenyl)methyl)amino)cyclohexyl)ethanol

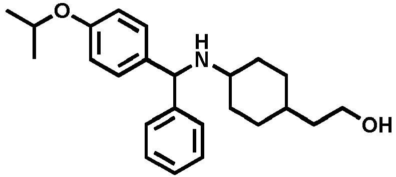

2-lodopropane (8.2ml, 82mmol) was added to a mixture of 4-hydroxyphenyl)(phenyl)methanone (5.42g, 27.3mmol) and K₂CO₃ (18.9g, 82mmol) in DMF (40ml) and the mixture stirred at 90 °C for 2h. At this time TLC showed complete consumption of 4-hydroxyphenyl)(phenyl)methanone. The mixture was partitioned between EA (200ml) and 0.1N sodium hydroxide (NaOH) (200ml) and the organic layer washed with saturated sodium chloride (NaCl) (200ml), dried with Na₂SO₄, filtered and the solvents removed in vacuo to give crude 4-isopropoxyphenyl)(phenyl)methanone which was purified by FC to give pure product as a pale yellow oil (6.5g, 99%). Sodium borohydride (NaBH₄) (692mg, 18.3mmol) was added portionwise to a 5 °C solution of 4-isopropoxyphenyl)(phenyl)methanone (4g, 16.6mmol) in methanol (MeOH) (30ml) and stirred at <15 °C for 1h. At this time TLC showed complete consumption of 4-isopropoxyphenyl)(phenyl)methanone. The mixture was partitioned between EA (100ml) and saturated NaCl (100ml) and the organic layer dried with Na₂SO₄, filtered and the solvents removed in vacuo to give 4-isopropoxyphenyl)(phenyl)methanol which was used for the next step without further purification (4.024g, 100%). Thionyl chloride (SOCl₂) (1.5ml, 20.6mmol) was added to a solution of 4-isopropoxyphenyl)(phenyl)methanol (1g, 4.1mmol) in chloroform (CHCl₃) (30ml), followed by DMF (64ul) and the solution stirred at room temperature for 3h. At this time TLC showed complete consumption of 4-isopropoxyphenyl)(phenyl)methanol. The solvents were removed in vacuo to give crude 1-(chloro(phenyl)methyl)-4-isopropoxybenzene which was used for the next step without further purification (1.08g, 100%). 2-(4-Aminocyclohexyl)ethanol (198mg, 1.4mmol) was added to a solution of 1-(chloro(phenyl)methyl)-4-isopropoxybenzene (300mg, 1.2mmol) in DMF (2ml), followed by diisopropylethylamine (DIPEA) (200ul, 1.2mmol) and the solution microwaved at 70 °C for 2h. At this time TLC showed complete consumption of 1-(chloro(phenyl)methyl)-4-isopropoxybenzene. The reaction mixture was partitioned between 15% isopropanol (IPA) in CHCl₃ (8ml) and 1N NaOH (8ml), the layers separated and the organic layer washed with saturated NaCl (8ml), dried with Na₂SO₄, filtered and the solvents removed in vacuo to give crude 2-(4-(((4-isopropoxyphenyl)(phenyl)methyl)amino)cyclohexyl)ethanol which was purified by FC to give pure product as a colorless oil (103mg, 24%). GC-MS showed >99% purity and desired mass (El ionization) M⁺ 367.5; ¹H NMR (60 MHz) δ 7.29-6.99 (7H, m), 6.75 (2H, d), 4.90 (1H, d), 4.46 (1H, m), 3.71-3.52 (2H, m), 2.91-2.66 (1H, m), 1.92-0.82 (20H, m).

### Example 3: N-((4-isopropoxyphenyl)(phenyl)methyl)-4-methylcyclohexanamine

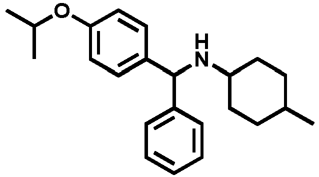

4-methylcyclohexylamine (91mg, 0.81mmol) was added to a solution of 1-(chloro(phenyl)methyl)-4-isopropoxybenzene (200mg, 0.76mmol) in THF (1ml), followed by TEA (111ul, 0.80mmol) and the solution stirred at R.T. for 24h. At this time TLC showed complete consumption of 1-(chloro(phenyl)methyl)-4-isopropoxybenzene. The reaction mixture was partitioned between 15% IPA in CHCl₃ (8ml) and 1N NaOH (8ml), the layers separated and the organic layer washed with saturated NaCl (8ml), dried with Na₂SO₄, filtered and the solvents removed in vacuo to give crude N-((4-isopropoxyphenyl)(phenyl)methyl)-4-methylcyclohexanamine which was purified by FC to give pure product as a colorless oil (164mg, 63%). GC-MS showed >98% purity and desired mass (El ionization) M+ 337.5.

### Melanoderm assay

Control compounds consisted of a negative control (ultrapure water), vehicle control [Dimethylsulfoxide (DMSO) in ultrapure water- final concentration of 0.1%] and positive control (2% kojic acid in ultrapure water-final concentration of 700uM). Test compounds where prepared in DMSO at 10mM concentrations and diluted with EPI-100-LLMM media (Mattek Corp.) to a final concentration of 10uM. Melanoderm human tissue (MEL-300-B, Mattek Corp.) was equilibrated with EPI-100-NMM media for 24h, followed by equilibration with EPI-100-LLMM media for 48h prior to treatment with test compounds and controls. Dosing and re-feeding of melanoderms with controls and test compounds continued for a total of 12d following standard protocols and the melanoderms collected for melanin quantitation, viability analysis and macroscopic/microscopic imaging analysis. For melanin quantitation, tissues were suspended in phosphate buffer saline (PBS) buffer (5ml) for 5min, removed from the PBS and washed/rinsed with additional PBS (2ml). Tissues were treated with 1% sodium bicarbonate solution (300ul) for 30min, followed by removal of the sodium bicarbonate solution and washed/rinsed with PBS solution (2 X 2ml). Tissues were suspended in Solvable reagent (500ul) and incubated at 95 °C for 24h along with melanin standard samples (prepared by suspending melanin (cat# M8631, Sigma-Aldrich) in Solvable reagent to generate melanin standards at 0, 2.5, 5, 10, 25, 50 and 100ug). All incubated samples were centrifuged at 13000rpm for 5min and the optical density of the supernatant was read/determined at 490nm. The amount of melanin (ug) from each tissue sample was determined from the melanin standard samples calibration curve. Protein concentration of tissue samples was determined using the BCA protein assay kit (Pierce) and melanin content was normalized and expressed as melanin (ug) / protein (ug) (Table 1). Tissue viability as a result of test compound dosing/feeding relative to negative and vehicle controls was determined using the WST-1 reagent kit (cat# 05015944001, Roche). Tissue images were examined macroscopically to assess the skin lightening ability of test compounds compared to negative, vehicle and positive controls and microscopically (10X magnification) to assess cytotoxicity.

### Melanoderm Data

**Table 1 Reduction in melanin content from melanoderm treatment with Compounds of example 1, 2 and 3.**

| Test Sample | Melanin (µg/µg protein)^{a} | P value^{b} |
|---|---|---|
| Vehicle control (0.1 vol% DMSO) | 0.240 +/- 0.020 | --- |
| Positive control (700µM) Kojic acid) | 0.128 +/- 0.004 | 0.0007^{c} |
| Compound of example 1 (10uM) | 0.158 +/- 0.006 | 0.0024^{d} |
| Compound of example 2 (10uM) | 0.076 +/- 0.012 | 0.0003^{e} |
| Compound of example 3 (10uM) | 0.144 +/- 0.006 | 0.0013^{f} |

| | | |
|---|---|---|
| ^{a} Mean +/- standard deviation from n = 3 samples. ^{b} T-test compared to vehicle control. ^{c} Statistically significant at P < 0.001. ^{d} Statistically significant at P < 0.003. ^{e} Statistically significant at P < 0.0005. ^{f} Statistically significant at P < 0.002. | | |

Compound of example 1, 2 and 3 showed reduced melanin content clearly showing the skin lightening effect.

## Claims

1. A compound of ,
formula 1a: 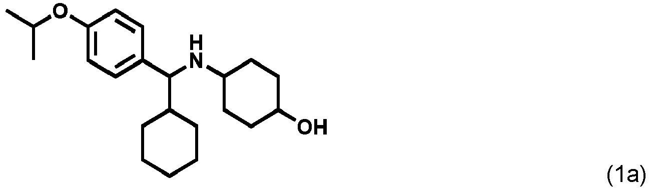 or formula 1b: 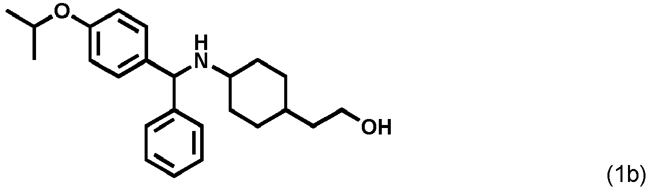 or formula 1c: 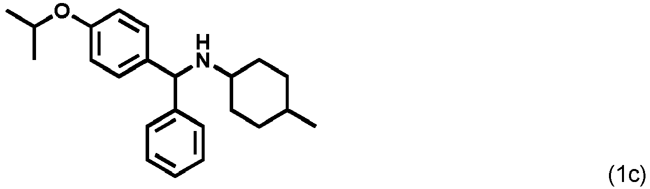 or cosmetically acceptable salt thereof.

2. A composition comprising
• 0.001 to 20 wt% of a compound according to claim 1 and
• a cosmetically acceptable base.

3. A composition according to claim 2 comprising 0.001 to 15 wt%, of at least one additional skin lightening agent selected from niacinamide, 12-hydroxystearic acid and resorcinol derivatives.

4. A composition according to claims 2 or 3 comprising 0.1 to 15 wt% of at least one organic sunscreen.

5. A composition according to any one of claims 2 to 4 comprising 0.1 to 15 wt% of at least one inorganic sunscreen.

6. A cosmetic method of lightening age spots and freckles comprising applying to the skin, a composition according to any one of claims 2 to 5.

7. A compound according to claim 1 for use in lightening age spots and freckles.

8. Use of a compound according to claim 1 as an agent for lightening age spots and freckles.

## Patentansprüche

1. Verbindung der Formel 1a: 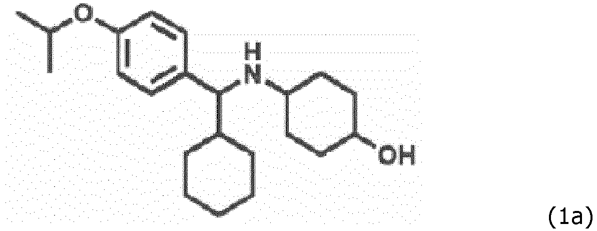 oder der Formel 1b: 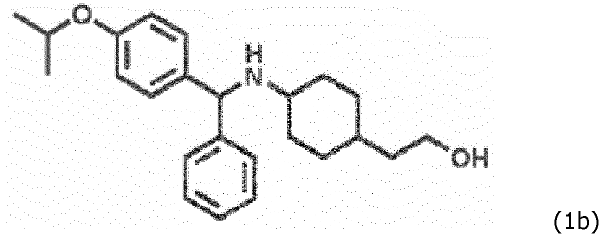 oder der Formel 1c: 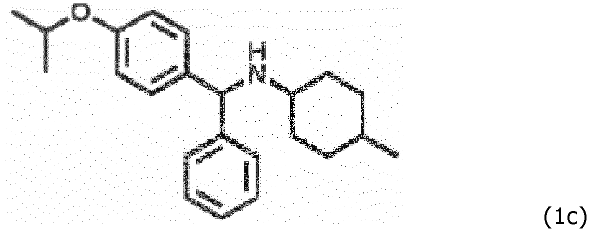 oder ein kosmetisch akzeptables Salz davon.

2. Zusammensetzung, umfassend
• 0,001 bis 20 Gew.-% einer Verbindung nach Anspruch 1 und
• eine kosmetisch akzeptable Grundlage.

3. Zusammensetzung nach Anspruch 2, umfassend 0,001 bis 15 Gew.-% mindestens eines zusätzlichen Hautaufhellungsmittels, ausgewählt unter Niacinamid, 12-Hydroxystearinsäure und Resorcin-Derivaten.

4. Zusammensetzung nach Anspruch 2 oder 3, umfassend 0,1 bis 15 Gew.-% mindestens eines organischen Sonnenschutzmittels.

5. Zusammensetzung nach irgendeinem der Ansprüche 2 bis 4, umfassend 0,1 bis 15 Gew.-% mindestens eines anorganischen Sonnenschutzmittels.

6. Kosmetisches Verfahren zum Aufhellen von Altersflecken und Sommersprossen, umfassend das Auftragen einer Zusammensetzung nach irgendeinem der Ansprüche 2 bis 5 auf die Haut.

7. Verbindung nach Anspruch 1 zur Verwendung beim Aufhellen von Altersflecken und Sommersprossen.

8. Verwendung einer Verbindung nach Anspruch 1 als Mittel zum Aufhellen von Altersflecken und Sommersprossen.

## Revendications

1. Composé de
formule 1a : 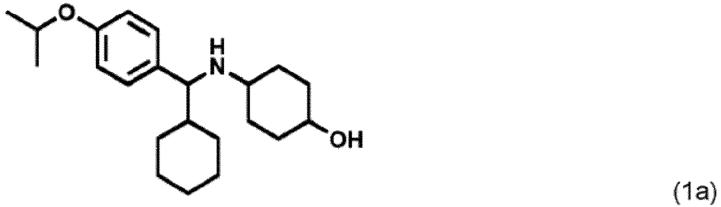 ou formule 1b : 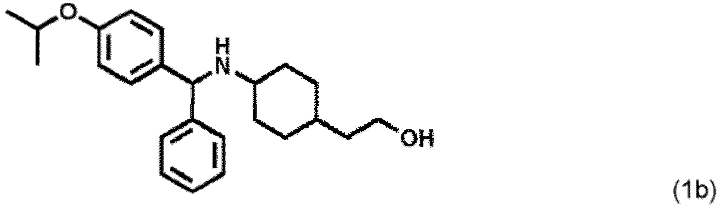 ou formule 1c : 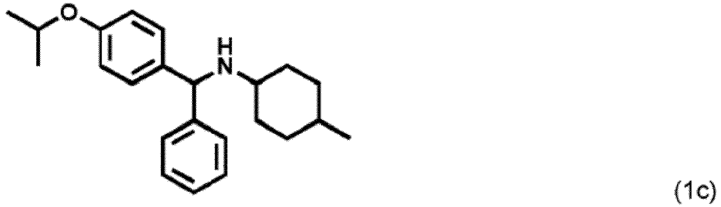 ou un sel cosmétiquement acceptable de celui-ci.

2. Composition comprenant
• de 0,001 à 20 % en poids d'un composé selon la revendication 1 et
• une base cosmétiquement acceptable.

3. Composition selon la revendication 2 comprenant de 0,001 à 15 % en poids d'au moins un agent d'éclaircissement de la peau supplémentaire choisi parmi la niacinamide, l'acide 12-hydroxystéarique et les dérivés du résorcinol.

4. Composition selon les revendications 2 ou 3 comprenant de 0,1 à 15 % en poids d'au moins un écran solaire organique.

5. Composition selon l'une quelconque des revendications 2 à 4, comprenant de 0,1 à 15 % en poids d'au moins un écran solaire inorganique.

6. Procédé cosmétique d'éclaircissement des taches de vieillesse et des taches de rousseur comprenant l'application sur la peau d'une composition selon l'une quelconque des revendications 2 à 5.

7. Composé selon la revendication 1 destiné à être utilisé pour l'éclaircissement des taches de vieillesse et des taches de rousseur.

8. Utilisation d'un composé selon la revendication 1 en tant qu'agent pour l'éclaircissement des taches de vieillesse et des taches de rousseur.
